# EUROPÄISCHE PATENTSCHRIFT

(11) **EP 0 317 810 B1**
(45) Veröffentlichungstag und Bekanntmachung des Hinweises auf die Patenterteilung: **24.08.1994**
(21) Anmeldenummer: 88118431.1
(22) Anmeldetag: 04.11.1988
(51) Int. Cl.: C12N 5/00, C12M 3/00

(54) **Verfahren und Vorrichtung zum Ablösen von Zellkulturen von Mikroträgern**
Process and apparatus for separating cell cultures from their microcarriers
Procédé et appareil pour séparer des cultures cellulaires de leurs microsupports

(30) Priorität: 23.11.1987 DE 3739649
(43) Veröffentlichungstag der Anmeldung: 31.05.1989
(73) Patentinhaber: IMMUNO Aktiengesellschaft, A-1221 Wien (AT)
(72) Erfinder: Mundt, Wolfgang, Dr., A-1080 Wien (AT)
(74) Vertreter: Grünecker, Kinkeldey, Stockmair & Schwanhäusser Anwaltssozietät

(56) Entgegenhaltungen:
- WO-A-86/01531
- DE-A- 2 300 567
- GB-A- 2 059 991
- US-A- 4 144 126

## Beschreibung

Die Erfindung betrifft ein Verfahren gemäß dem Oberbegriff des Anspruches 1.

Das Verfahren dient der Gewinnung von Zellkulturen durch deren Vermehrung auf Mikroträgern. Um Zellkulturen in größerer Menge zu erhalten ist es notwendig, die Kulturen jeweils von den Mikroträgern zu lösen und auf unbesetzte Mikroträger zu übersiedeln. Bisher wird dabei schubweise (batch-weise) gearbeitet. Einem Behälter mit besiedelten Mikroträgern wird Trypsin zugeführt, das die Zellen zum Ablösen veranlaßt. Mit der Entnahme der Zellen, d.h. einer die Zellen enthaltenden Zellsuspension, aus dem Behälter wird gewartet, bis im wesentlichen alle Zellen von den Mikroträgern abgelost sind. Ein hoher Prozentsatz der Zellen löst sich ziemlich schnell und verweilt dadurch lange Zeit in der Trypsinlösung. Dies hat den Nachteil, daß die Einwirkung von Trypsin diese Zellen ungünstig beeinflußt. Insbesondere wird ihr Wachstum und ihr Ansiedlungsverhalten auf neuen Mikroträgern beeinträchtigt. Dies wird besonders kritisch bei größeren Mengen, wo die Kulturen inhomogen sind und mit sehr unterschiedlichen Ablösegeschwindigkeiten gerechnet werden muß.

Aufgabe der Erfindung ist es, ein Verfahren der eingangs beschriebenen Art zu schaffen, bei welchem die Einwirkzeiten des Trypsins auf abgelöste Zellen auf ein Minimum beschränkt wird.

Die Aufgabe wird erfindungsgemäß durch ein Verfahren nach dem kennzeichnenden Teil des Anspruches 1 gelöst.

Abgelöste Zellen werden dabei in einer der Durchflußgeschwindigkeit der Trypsinlösung entsprechenden Geschwindigkeit aus dem Behälter entfernt und dann der schädigenden Einwirkung entzogen. Der Durchfluß der Trypsinlösung kann solange fortgesetzt werden wie nötig, um auch die letzten Zellkulturen von den Mikroträgern zu trennen. Die Einwirkungszeit des Trypsins auf abgelöste Zellen ist in jedem Stadium etwa gleich kurz.

Der Durchfluß der Trypsinlösung gemäß Anspruch 2 hat den Vorteil, daß sich keine abgelösten Zellen im Bodenbereich des Behälters absetzen können und im Zuge der Strömungsrichtung auch aus dem Bereich der Mikroträger herausgefördert werden.

Das Merkmal des Anspruches 3 spricht eine vorteilhafte Verfahrensform an, bei welcher die Mikroträger allseitig von der durchfließenden Lösung umspült werden und das Ablösen beschleunigt wird.

Die Verfahrensform nach Anspruch 4 hat den Vorteil, daß die Trypsinlösung gleichmäßig verteilt und mit homogener Geschwindigkeitsverteilung auf die Mikroträger und ihre Zellkulturen trifft.

Vorteilhaft werden die abgelösten Zellen gemäß Anspruch 5 in einen anderen, leere Mikroträger enthaltenden Behälter weitergeführt, wo die weitere Vermehrung sofort beginnen kann.

Die Erfindung schafft auch eine Vorrichtung zur Durchführung des Verfahrens gemäß Anspruch 7, wodurch der Durchfluß der Trypsinlösung ermöglicht ist.

Eine vorteilhafte Ausführungsform gemäß Anspruch 8 ermöglicht den Durchfluß einer Waschpufferflüssigkeit von oben nach unten durch den Behälter vor der Trypsineinführung.

Die Aufbereitung des benutzten Behälters und der benutzten Mikroträger für die erneute Verwendung wird vorteilhaft durch das Merkmal des Anspruches 9 erleichtert.

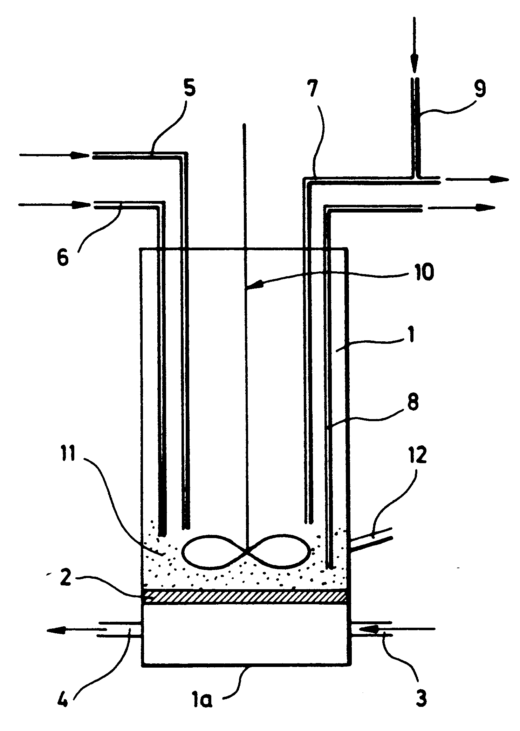

Das erfindungsgemäße Verfahren wird nachstehend anhand einer schematisch dargestellten Vorrichtung (einzige Figur) erläutert.

In eine geschlossenen Behälter 1 befindet sich im Abstand über dessen Boden 1a ein durchlässiger Einsatz 2, z.B. ein Sieb oder eine poröse Platte. Unterhalb des Einsatzes 2 mündet in den Behälter eine Zuflußleitung 3 für das Einführen einer Trypsinlösung. Ebenfalls im Wandbereich des Behälters zwischen dem Boden 1a und dem Einsatz 2 befindet sich eine verschließbare Ausflußöffnung 4 für den Abfluß einer Waschpufferflüssigkeit. Oberhalb des durchlässigen Einsatzes 2 münden in den Behälter 1 ein Zufluß 5 für eine Waschpufferflüssigkeit und ein Zulauf 6 für ein Medium zur Kulturvermehrung. Ferner ragt von oben in den Behälter eine Entnahmeleitung 7 für eine gelöste Zellen enthaltende Suspension und eine Ablaufleitung 8, wobei die Eintrittsöffnung der letzteren sich dicht über dem Einsatz 2 befindet. In die Entnahmeleitung 7 mündet außerhalb des Behälters eine Zuflußleitung 9. Ferner ist in der Entnahmeleitung 7 eine hier nicht dargestellte Absaugpumpe angeordnet.

Ferner reicht in den Behälter von oben ein Rührorgan 10, das auf nicht dargestellte Weise außerhalb des Behälters antreibbar gelagert ist.

Oberhalb des durchlässigen Einsatzes 2 befindet sich eine durch Punkte angedeutete, beliebig hohe Schicht von Mikroträgern 11. Auf diesen haben sich vor dem Beginn des zu schildernden Verfahrens Zellkulturen angesiedelt und vermehrt, bis die Mikroträger 11 jeweils vollständig besetzt sind. Dieses Stadium kann durch eine Probeentnahme an einer Anzapfstelle 12 oberhalb des Einsatzes 2 festgestellt werden. Dann wird zunächst im Durchflußverfahren eine Waschpufferlösung durch den Behälterinhalt geleitet, die durch den Zufluß 5 eintritt und durch die gleichzeitig geöffnete Ausflußöffnung 4 abfließt. Nachdem der Ausfluß 4 geschlossen ist, wird durch die Zuflußleitung 3 eine Trypsinlösung in den Behälter gepumpt. Sie wird durch den durchlässigen Einsatz 2 hindurch verteilt und mit leichtem, gleichmäßigen Strömungsdruck auf die Mikroträger 11 gerichtet, wodurch diese in der Schwebe gehalten werden. Unter der Einwirkung des Trypsins lösen sich die Zellkulturen von den Mikroträgern ab. Die Trypsinlösung wird im Durchflußverfahren über die Entnahmeleitung 7 mittels einer Absaugpumpe aus dem Behälter herausgeführt. Abgelöste Zellen werden auf diesem Weg sofort mitgenommen und aus dem Behälter entfernt. Durch die Zuflußleitung 9 wird gleichzeitig ein Medium in die Entnahmeleitung 7 eingebracht, welches das Trypsin inaktiviert und/oder ausscheidet. Damit werden die ausgeschwemmten Zellen von weiterer Einwirkung des Trypsins befreit. Sie können dann direkt einem anderen Behälter mit unbesetzten Mikroträgern zugeführt werden.

Der Durchfluß der Trypsinlösung wird so lange aufrechterhalten, bis im wesentlichen alle Zellkulturen abgelöst und aus dem Behälter ausgetragen sind.

Die Durchflußrate der Trypsinlösung wird dabei so bemessen, daß sich die Mikroträger 11 in der Schwebe befinden, wodurch sie für die Trypsinlösung allseitig zugänglich sind und abgelöste Zellen rasch wegbefördert werden.

Nach Abschluß der Zellkulturentnahme werden die im wesentlichen leeren Mikroträger über die Ablaufleitung 8 aus dem Behälter entnommen. Sie werden dann für eine Neubesiedlung vorbereitet, ebenso wie der Behälter 1.

Das Rührwerk 10 kann bei Bedarf zum schnelleren Durchmischen der Mikroträger mit der Waschpufferlösung bei Bedarf betätigt werden.

## Patentansprüche

1. Verfahren zum Ablösen von Zellkulturen von Mikroträgern, bei dem einem Behälter mit besiedelten Trägern eine Trypsinlösung zugeführt wird und die abgelösten Zellen aus dem Behälter abgeführt werden,
**dadurch gekennzeichnet,**
daß die Trypsinlösung im Durchflußverfahren durch den Behälter und damit durch die Mikroträger geführt und abgelöste Zellen somit gleich aus dem Behälter ausgeschwemmt werden, wobei die Trypsinlösung nach dem Verlassen des Behälters inaktiviert und/oder ausgeschieden wird.

2. Verfahren nach Anspruch 1,
**dadurch gekennzeichnet,**
daß die Trypsinlösung von unten nach oben durch den Behälter gepumpt wird.

3. Verfahren nach Anspruch 2,
**dadurch gekennzeichnet,**
daß die Durchflußrate der Trypsinlösung derart bemessen wird, daß die Mikroträger in der Schwebe gehalten werden.

4. Verfahren nach wenigstens einem der Ansprüche 1 bis 3,
**dadurch gekennzeichnet,**
daß die Trypsinlösung dem die Mikroträger enthaltenden Behälterbereich von unten durch einen durchlässigen Einsatz zugeführt wird.

5. Verfahren nach wenigstens einem der Ansprüche 1 bes 4,
**dadurch gekennzeichnet,**
daß die abgelösten Zellkulturen zu einem leere Mikroträger enthaltenden Behälter weitergeführt werden.

6. Verfahren nach wenigstens einem der Ansprüche 1 bes 5,
**dadurch gekennzeichnet,**
daß durch den Behälter mit besiedelten Mikroträgern vor dem Einführen der Trypsinlösung eine Waschpufferflüssigkeit im Durchflußverfahren geleitet wird.

7. Vorrichtung zum Durchführen des Verfahrens zu einem der Ansprüche 1 bis 6,
**gekennzeichnet durch**
einen Behälter (1) zur Aufnahme von Mikroträgern (11), der in Abstand von seinem Boden (1a) einen durchlässigen Einsatz (2) enthält, und bei dem die Zuflußleitung (3) für die Trypsinlösung unterhalb des Einsatzes (2) einmündet, und die Entnahmeleitung (7) für eine abgelöste Zellen enthaltende Suspension im Behälter oberhalb des durchlässigen Einsatzes (2) beginnt, wobei außerhalb des Behälters (1) in die Entnahmeleitung (7) eine Zuflußleitung (9) für ein die Trypsinlösung inaktivierendes Medium und/oder zum Abscheiden von Trypsin mündet.

8. Vorrichtung nach Anpruch 7,
**dadurch gekennzeichnet,**
daß in den oberen Behälterbereich ein Zufluß (5) für eine Waschpufferflüssigkeit mündet und im Bodenbereich unter dem durchlässigen Einsatz (2) eine verschließbare Ausflußöffnung (4) angeordnet ist.

9. Vorrichtung nach Anspruch 7 oder 8,
**dadurch gekennzeichnet,**
daß eine Ablaufleitung (8) zum Entfernen von Mikroträgern (11) aus dem Behälter (1) sich von dicht über dem Einsatz (2) bis oben aus dem Behälter (1) herauserstreckt.

10. Vorrichtung nach einem der Ansprüche 7 bis 9,
**dadurch gekennzeichnet,**
daß der Behälter (1) oberhalb des durchlässigen Einsatzes (2) eine Anzapfstelle (12) für eine Probeentnahme von Mikroträgern aufweist.

## Claims

1. Process for detaching cell cultures from microcarriers, in which a trypsin solution is conveyed to a vessel containing colonised carriers, and the detached cells are conveyed out of the vessel,
**characterised in that,**
in a through-flow process, the trypsin solution is conveyed through the vessel and accordingly through the microcarriers, and detached cells are thus immediately flushed out of the vessel, the trypsin solution being inactivated and/or separated off after leaving the vessel.

2. Process according to claim 1,
**characterised in that**
the trypsin solution is pumped through the vessel from the bottom to the top.

3. Process according to claim 2,
**characterised in that**
the flow rate of the trypsin solution is such that the microcarriers are maintained in suspension.

4. Process according to at least one of claims 1 to 3,
**characterised in that**
the trypsin solution is fed from below through a permeable insert to the region of the vessel containing the microcarriers.

5. Process according to at least one of claims 1 to 4,
**characterised in that**
the detached cell cultures are conveyed further to a vessel containing empty microcarriers.

6. Process according to at least one of claims 1 to 5,
**characterised in that,**
in a through-flow process, a washing buffer liquid is conveyed through the vessel containing colonised microcarriers before the trypsin solution is introduced.

7. Apparatus for carrying out the process of any one of claims 1 to 6,
**characterised by**
a vessel (1) for accommodating microcarriers (11) that has a permeable insert (2) at a distance from its base (1a), and in the case of which vessel the feed tube (3) for the trypsin solution opens out below the insert (2), and the removal tube (7) for a suspension containing detached cells begins in the vessel at a site above the permeable insert (2), a feed tube (9) for a medium that inactivates the trypsin solution and/or for separating trypsin leading into the removal tube (7) outside the vessel (1).

8. Apparatus according to claim 7,
**characterised in that**
a feed tube (5) for a washing buffer liquid opens out in the upper region of the vessel, and a closable outlet opening (4) is arranged in the base region below the permeable insert (2).

9. Apparatus according to claim 7 or claim 8,
**characterised in that**
a discharge tube (8) for removing microcarriers (11) from the vessel (1) extends from immediately above the insert (2) and out of the top of the vessel (1).

10. Apparatus according to any one of claims 7 to 9,
**characterised in that**
the vessel (1) has, above the permeable insert (2), a tapping point (12) for removing a sample of the microcarriers.

## Revendications

1. Procédé pour détacher des cultures cellulaires d'avec des microsupports, dans lequel on amène dans un récipient contenant des supports colonisés une solution de trypsine et on évacue hors du récipient les cellules détachées, caractérisé par le fait que, dans le procédé de circulation, la solution de trypsine est conduite à travers le récipient et donc à travers les microsupports et que les cellules détachées sont aussitôt emportées hors du récipient, la solution de trypsine étant inactivée et/ou éliminée après avoir quitté le récipient.

2. Procédé selon la revendication 1, caractérisé par le fait que la solution de trypsine est pompée à travers le récipient de bas en haut.

3. Procédé selon la revendication 2, caractérisé par le fait que le débit de circulation de la solution de trypsine est dosé de façon que les microsupports soient maintenus en suspension.

4. Procédé selon au moins l'une des revendications 1 à 3, caractérisé par le fait que c'est d'en bas, à travers une garniture perméable, que la solution de trypsine est amenée à la zone du récipient contenant les microsupports.

5. Procédé selon au moins l'une des revendications 1 à 4, caractérisé par le fait que les cultures cellulaires détachées sont dirigées vers un récipient contenant des microsupports vides.

6. Procédé selon au moins l'une des revendications 1 à 5, caractérisé par le fait qu'avant d'introduire la solution de trypsine un liquide tampon de lavage est conduit dans le procédé de circulation à travers le récipient contenant les microsupports colonisés.

7. Dispositif pour la mise en oeuvre du procédé selon l'une des revendications 1 à 6, caractérisé par un récipient (1) pour la réception de microsupports (11) qui, à une certaine distance de son fond (1a), contient une garniture perméable (2) et dans le cas duquel la conduite d'arrivée (3) pour la solution de trypsine débouche en-dessous de la garniture (2) et la conduite (7) de prélèvement dans le récipient d'une suspension contenant des cellules détachées commence au-dessus de la garniture perméable (2), étant précisé qu'à l'extérieur du récipient (1) débouche dans la conduite de prélèvement (7) une conduite d'arrivée (9) pour un milieu inactivant la solution de trypsine et/ou pour éliminer la trypsine.

8. Dispositif selon la revendication 7, caractérisé par le fait que dans la zone supérieure du récipient débouche une arrivée (5) pour un liquide tampon de lavage et que dans la zone du fond, en-dessous de la garniture perméable (2), est ménagée une ouverture de sortie obturable (4).

9. Dispositif selon la revendication 7 ou 8, caractérisé par le fait qu'une conduite de départ (8) pour éloigner des microsupports (11) hors du récipient (1) s'étend depuis très près au-dessus de la garniture (2) jusqu'en haut hors du récipient (1).

10. Dispositif selon l'une des revendications 7 à 9, caractérisé par le fait que le récipient (1) présente, au-dessus de la garniture perméable (2), une position de soutirage (12) pour un prélèvement d'échantillons de microsupports.
